# EUROPEAN PATENT SPECIFICATION

(11) **EP 3 785 614 B1**
(45) Date of publication and mention of the grant of the patent: **09.11.2022**
(21) Application number: 20199877.0
(22) Date of filing: 21.03.2019
(51) Int. Cl.: A61B 5/00, A61B 5/021, A61B 5/08, A61B 3/11

(54) **MEASUREMENT OF PHYSIOLOGICAL PARAMETERS**
MESSUNG VON PHYSIOLOGISCHEN PARAMETERN
MESURE DE PARAMÈTRES PHYSIOLOGIQUES

(30) Priority: 26.03.2018 US 201862648030 P; 26.03.2018 US 201862648041 P; 26.03.2018 US 201862648054 P
(43) Date of publication of application: 03.03.2021
(62) Divisional of application: 19722679.8
(73) Proprietor: Diagnostic Robotics Ltd., 6744320 Tel Aviv (IL)
(72) Inventor: GLOZMAN, Daniel, 4034723 Kfar Yona (IL); AMIR, Yonatan, 9310503 Jerusalem (IL)
(74) Representative: Boult Wade Tennant LLP

(56) References cited:
- US-A- 5 196 872
- US-A- 6 095 989
- US-A1- 2012 008 091
- US-A1- 2013 250 244

## Description

### FIELD OF EMBODIMENTS OF THE INVENTION

The present invention relates to methods and apparatus for measuring a pupillary response.

### BACKGROUND

Respiratory rate is a clinical parameter that is of significant importance in assessing a patient's condition, and is considered one of the vital signs, along with blood pressure, heart rate, oxygen saturation, and body temperature. Many disorders can be diagnosed at least partially on the basis of an abnormal respiratory rate. For example, an abnormal respiratory rate may be indicative of asthma, chronic obstructive pulmonary disease, acute respiratory distress syndrome, emphysema, congestive heart failure, etc. Respiratory rate may also increase with fever, illness, and with other medical conditions

Respiratory rate is usually measured when a patient is at rest and involves counting the number of breaths that the patient breathes over the course of one minute, by counting how many times the chest rises.

Blood pressure is the pressure of circulating blood on the walls of blood vessels. Blood pressure is a clinical parameter that is of significant importance in assessing a patient's condition, and is considered one of the vital signs, as described above. Blood pressure generally refers to the arterial pressure in the systemic circulation. The blood pressure in the systemic circulation is also referred to as systemic blood pressure.

Arterial pressure is most commonly measured via a sphygmomanometer, which historically used the height of a column of mercury to reflect the circulating pressure. Blood pressure values are generally reported in millimeters of mercury (mmHg). The auscultatory method of measuring blood pressure uses a stethoscope and a sphygmomanometer. A cuff is placed around the upper arm at roughly the same vertical height as the heart. A manometer (which is typically a mercury manometer), measures the height of a column of mercury, giving an absolute result without need for calibration. The oscillometric method of measuring blood pressure also uses a sphygmomanometer cuff, but an electronic pressure sensor is used to observe cuff pressure oscillations.

Arterial blood pressure varies with the height of the measuring device above or below the heart. *Ceteris paribus,* when the body is upright, the lower in the body a measurement is made, the higher the measured blood pressure, due to the fact that there is a greater volume of blood that is exerting its weight upon the blood. In order to provide a standardized measure of systemic blood pressure, systemic blood pressure is typically measured at heart height.

The pupillary light reflex is a reflex that controls the diameter of the pupil, in response to the intensity of light that falls on the retina in the back of the eye. An increase in intensity of light shone into even one of the eyes causes the pupils of both eyes to constrict, whereas a decrease in the intensity of the light causes the pupils of both eyes to dilate.

The pupillary light reflex provides a useful diagnostic tool. It allows for testing the integrity of the sensory and motor functions of the eye. Emergency room physicians routinely assess the pupillary reflex because it is useful for assessing brain stem function. Normally, pupils react equally. Lack of the pupillary reflex or an abnormal pupillary reflex can be caused by optic nerve damage, oculomotor nerve damage, brain stem death and/or drugs.

### SUMMARY OF EMBODIMENTS

In accordance with some applications of the present disclosure, (but not part of the claimed invention), a patient's respiratory rate is automatically measured. For some applications, a surface is configured to receive the patient's arm. A sensor that is operatively coupled to the surface, detects movement of the surface, pressure exerted upon the surface, and/or force exerted upon the surface, and generates a sensor signal in response thereto. Typically, due motion of the patient's arm resulting from the patient's respiratory cycle, the sensor signal contains a cyclical component that corresponds to the patient's respiratory cycle. A computer processor is configured to receive the sensor signal, and to derive the patient's respiratory rate, at least partially based upon the received sensor signal.

For some applications, the computer processor derives the patient's respiratory cycle from the sensor signal by identifying a cyclical component within the signal and determining a parameter of the cyclical component, such as the mean frequency of the cyclical component, the mean period of the cyclical component, and/or the number of occurrences of the cyclical component over a given time interval (e.g., over the course of a minute). For some applications, the computer processor filters the sensor signal, in order to identify the cyclical component. For example, the computer processor may be configured to identify the cyclical component, by identifying a cyclical component having a minimum period of between 1 second and 3 seconds, and/or a maximum period of between 15 seconds and 30 seconds.

It is noted that even if the amplitude of the cyclical component of the sensor signal as described hereinabove is low, the computer processor is typically configured to detect the cyclical component, e.g., by identifying a component of the sensor signal that is cyclical and that has a frequency within a given range, as described hereinabove. For some applications, the computer processor is further configured to determine additional parameters of the patient's respiratory cycle, by analyzing the cyclical component of the sensor signal described hereinabove. For example, the computer processor may be configured to determine a ratio (e.g., a mean ratio) between the duration of the patient's inspiration and the duration of the patient's expiration within the patient's respiratory cycle.

In accordance with some applications of the present disclosure, (but not part of the claimed invention), a patient-testing station includes apparatus for measuring a patient's systemic blood pressure. Typically, the patient-testing station includes pressure-sensing apparatus, which is placed in contact with a portion of the patient's body, e.g., the patient's wrist. For some applications, the pressure-sensing apparatus includes a compressible portion (e.g., an inflatable cuff, or sleeve) configured to be placed in contact with the portion of the patient's body, and a pressure sensor configured to measure blood pressure of the portion of the patient's body, by detecting pressure applied to the compressible portion by the portion of the patient's body. A camera is typically configured to acquire one or more images of the patient. For some applications, at least one computer processor estimates a location of patient's heart, by analyzing the one or more images of the patient.

The computer processor typically estimates a difference in height between the portion of the patient's body that is in contact with the pressure-sensing apparatus (e.g., the patient's wrist) and the estimated location of the patient's heart, and generates an output in response thereto. Typically, the computer processor determines the patient's systemic blood pressure, based upon the blood pressure of the portion of the patient's body (e.g., the patient's wrist) measured by the pressure-sensing apparatus, and the estimated difference in height between the portion of the patient's body that is in contact with the pressure-sensing apparatus and the estimated location of the patient's heart. For example, the computer processor may apply a compensation to the pressure measured by the pressure-sensing apparatus, to account for the estimated difference in height between the portion of the patient's body that is in contact with the pressure-sensing apparatus and the estimated height of the patient's heart.

In accordance with the present invention, a patient's pupillary light reflex is measured automatically as defined in claim 4. At least one image-acquisition device acquires a plurality of images of at least a portion of the patient's face. For example, the image-acquisition device may be a video camera that is configured to acquire images of at least a portion of the patient's face that includes at least one of the patient's eyes. At least one computer processor identifies a first eye of the patient within at least a first portion of the acquired images. For some applications, the computer processor identifies the pupil of the first eye within first portion of the acquired images. In response to identifying the first eye (and/or the pupil thereof), the computer drives a moveable light source (e.g., a laser and/or a broadband light) to direct light toward the patient's second eye (and the pupil thereof). For some applications, the moveable light source is configured to be moved automatically, and the movability of the light source typically is in at least two degrees of freedom, such that light from the light source can be directed anywhere upon the patient's face.

The computer processor measures the pupillary light reflex of the second eye to the light being directed toward the first eye, by identifying a pupil of the patient's first eye in images belonging to the first portion of the acquired images that were acquired, respectively, prior to and subsequent to the light being directed toward the first eye. For example, in a first image that was acquired prior to light being directed toward the patient's left eye, the computer processor may identify the pupil of the patient's left eye and may determine that the pupil has a diameter of x mm. The computer processor may then identify the pupil of the patient's left eye within images that were acquired subsequent to the light being directed toward the patient's right eye, and may thereby determine in which of those images the diameter of the pupil has decreased relative to x mm, and/or in which of those images the diameter of the pupil has decreased by more than a threshold amount and/or more than a threshold percentage, relative to x mm.

The computer processor is configured to identify the pupil of the patient's second eye within at least some of the acquired images. By way of example, the computer processor may be configured to identify the pupil of the patient's right eye within some of the acquired images. The computer processor measures the patient's consensual pupillary light reflex, by measuring a pupillary light reflex of the second eye, to the light being directed toward the first eye. The computer processor does so by identifying the pupil of the patient's second eye in images that were acquired, respectively, prior to and subsequent to the light being directed toward the first eye. For example, in a first image that was acquired prior to the light being directed toward the patient's left eye, the computer processor may identify the pupil of the patient's right eye and may determine that the pupil has a diameter of x mm. The computer processor may then identify the pupil of the patient's right eye within images that were acquired subsequent to the light being directed toward the patient's left eye, and may thereby determine in which of those images the diameter of the pupil has decreased relative to x mm, and/or in which of those images the diameter of the pupil has decreased by more than a threshold amount and/or more than a threshold percentage, relative to x mm.

For some applications (but not part of the claimed invention), the computer processor diagnoses a condition of the patient, generates an alert, and/or generates a different output at least partially based upon the pupillary light reflex of the first eye, and/or the second eye.

For some applications, the computer processor determines the patient's pupillary light reflex in a generally similar manner to that described hereinabove. However, rather than identifying the patient's first eye (and/or the pupil thereof) and directing light toward the first eye (and/or the pupil thereof), the computer processor drives the light source to generate a flash of light that is not specifically directed toward the patient's first eye (and/or the pupil thereof). The computer processor identifies pupils of the patient's first eye and/or second eye in images acquired, respectively, before and after the generation of the flash of light, and thereby determines the patient's pupillary light reflex in a generally similar manner to that described hereinabove.

There is therefore provided (but not part of the claimed invention) an apparatus including:
a surface configured to receive an arm of a patient;
a first sensor operatively coupled to the surface and configured to (a) detect a parameter selected from the group consisting of: movement of the surface, pressure exerted upon the surface, and force exerted upon the surface, and (b) generate a first sensor signal in response thereto; and
at least one computer processor configured to receive the first sensor signal, and to derive a respiratory rate of the patient at least partially based upon the received first sensor signal.

In some applications, the surface is hingedly coupled to a supporting element, via a hinge, such that when the patient's arm is disposed upon the surface, the surface moves as a result of movement of the patient's arm.

In some applications, the apparatus is for use with a chair upon which the patient sits, the apparatus further including:
a compressible structure disposed upon the chair; and
a second sensor operatively coupled to the compressible structure, the second sensor configured to (a) detect a parameter selected from the group consisting of: movement of the compressible structure, pressure exerted upon the compressible structure, and force exerted upon the compressible structure, and (b) generate a second sensor signal in response thereto,
the at least one computer processor being configured to receive the second sensor signal, and to derive the patient's respiratory rate at least partially based upon the received first and second sensor signal.

In some applications, the computer processor is configured to derive the patient's respiratory rate at least partially by identifying a cyclical component within the first sensor signal.

In some applications, the computer processor is configured to derive the patient's respiratory rate at least partially by determining a parameter of the cyclical component selected from the group consisting of: a mean frequency of the cyclical component, a mean period of the cyclical component, and number of occurrences of the cyclical component over a given time interval.

In some applications, the computer processor is configured to identify the cyclical component, by identifying a cyclical component having a minimum period of between 1 second and 3 seconds.

In some applications, the computer processor is configured to identify the cyclical component, by identifying a cyclical component having a maximum period of between 15 second and 30 seconds.

In some applications, the computer processor is further configured to determine a ratio between a duration of inspiration and a duration of expiration within a respiratory cycle of the patient, by analyzing the cyclical component.

There is further provided (but not part of the claimed invention), a method for use with a surface configured to receive an arm of a patient, the method including:
using a first sensor that is operatively coupled to the surface:
   detecting a parameter selected from the group consisting of: movement of the surface, pressure exerted upon the surface, and force exerted upon the surface; and
   generating a first sensor signal in response thereto; and
using at least one computer processor:
   receiving the first sensor signal; and
   deriving a respiratory rate of the patient at least partially based upon the received first sensor signal.

In some applications,
the method is for use with a chair upon which the patient sits and a compressible structure disposed upon the chair, the method further including:
using a second sensor that is operatively coupled to the compressible structure:
   detecting a parameter selected from the group consisting of: movement of the compressible structure, pressure exerted upon the compressible structure, and force exerted upon the compressible structure; and
   generating a second sensor signal in response thereto; and
using the at least one computer processor receiving the second sensor signal, deriving the patient's respiratory rate including deriving the patient's respiratory rate at least partially based upon the received first and second sensor signal.

In some applications, deriving the patient's respiratory rate includes deriving the patient's respiratory rate at least partially by identifying a cyclical component within the first sensor signal.

In some applications, deriving the patient's respiratory rate includes deriving the patient's respiratory rate at least partially by determining a parameter of the cyclical component selected from the group consisting of: a mean frequency of the cyclical component, a mean period of the cyclical component, and number of occurrences of the cyclical component over a given time interval.

In some applications, identifying the cyclical component includes identifying a cyclical component having a minimum period of between 1 second and 3 seconds.

In some applications, identifying the cyclical component includes identifying a cyclical component having a maximum period of between 15 second and 30 seconds.

In some applications, the method further includes, using the at least one computer processor, determining a ratio between a duration of inspiration and a duration of expiration within a respiratory cycle of the patient, by analyzing the cyclical component.

There is further provided (but not part of the claimed invention), an apparatus configured to sense systemic blood pressure of a patient, the apparatus including:
pressure-sensing apparatus configured to be placed in contact with a portion of a body of the patient;
a camera configured to acquire one or more images of the patient; and
at least one computer processor configured to:
   estimate a location of a heart of the patient, by analyzing the one or more images of the patient;
   estimate a difference in height between the portion of the patient's body that is in contact with the pressure-sensing apparatus and the estimated location of the patient's heart; and
   generate an output, at least partially in response thereto.

In some applications, the pressure-sensing apparatus includes a compressible portion configured to be placed in contact with the portion of the patient's body, and a pressure sensor configured to measure blood pressure of the portion of the patient's body by detecting pressure applied to the compressible portion by the portion of the patient's body.

In some applications, the apparatus further includes a surface having markings thereon and configured to be disposed in a vicinity of the patient during acquisition of the one or more images of the patient, and the computer processor is configured to estimate the location of the patient's heart by identifying at least some of the markings within the one or more images of the patient.

In some applications, the apparatus further includes a surface having markings thereon and configured to be disposed in a vicinity of the patient during acquisition of the one or more images of the patient, and the computer processor is configured to estimate the difference in height between the portion of the patient's body that is in contact with the pressure-sensing apparatus and the estimated location of the patient's heart, by identifying at least some of the markings within the one or more images of the patient.

In some applications, the at least one computer processor is configured to estimate the patient's systemic blood pressure by applying a compensation to the pressure measured by the pressure-sensing apparatus, to account for the estimated difference in height between the portion of the patient's body that is in contact with the pressure-sensing apparatus and the estimated height of the patient's heart.

In some applications, the at least one computer processor is configured to generate the output, by automatically reducing a height difference between at least a portion of the pressure-sensing apparatus and the estimated location of the heart.

In some applications, the apparatus is configured to be used with a chair upon which the patient sits, and the at least one computer processor is configured to automatically reduce the height difference between the portion of the pressure-sensing apparatus and the estimated location of the heart, by automatically adjusting a height of the chair.

In some applications, the at least one computer processor is configured to automatically reduce the height difference between the portion of the pressure-sensing apparatus and the estimated location of the heart, by automatically adjusting a height of the portion of the pressure-sensing apparatus.

There is provided (but not part of the claimed invention), a method for sensing systemic blood pressure of a patient, the method being for use with pressure-sensing apparatus configured to be placed in contact with a portion of a body of the patient and an output device, the method including:
acquiring one or more images of the patient, using a camera; and using at least one computer processor:
estimating a location of a heart of the patient, by analyzing the one or more images of the patient;
estimating a difference in height between the portion of the patient's body that is in contact with the pressure-sensing apparatus and the estimated location of the patient's heart; and
generate an output on the output device, at least partially in response thereto.

In some applications, the method is for use with a surface having markings thereon and configured to be disposed in a vicinity of the patient during acquisition of the one or more images of the patient, and estimating the location of the patient's heart includes identifying at least some of the markings within the one or more images of the patient.

In some applications, the method is for use with a surface having markings thereon and configured to be disposed in a vicinity of the patient during acquisition of the one or more images of the patient, and estimating the difference in height between the portion of the patient's body that is in contact with the pressure-sensing apparatus and the estimated location of the patient's heart includes identifying at least some of the markings within the one or more images of the patient.

In some applications, estimating the patient's systemic blood pressure includes applying a compensation to the pressure measured by the pressure-sensing apparatus, to account for the estimated difference in height between the portion of the patient's body that is in contact with the pressure-sensing apparatus and the estimated height of the patient's heart.

In some applications, generating the output includes automatically reducing a height difference between at least a portion of the pressure-sensing apparatus and the estimated location of the heart.

In some applications, the method is configured to be used with a chair upon which the patient sits, and the at least one computer processor is automatically reducing the height difference between the portion of the pressure-sensing apparatus and the estimated location of the heart includes automatically adjusting a height of the chair.

In some applications, automatically reducing the height difference between the portion of the pressure-sensing apparatus and the estimated location of the heart includes automatically adjusting a height of the portion of the pressure-sensing apparatus.

There is further provided, in accordance with the invention, an apparatus according to claim 1.

For some applications, the at least one computer processor is configured to identify the patient's first eye within at least the first portion of the acquired images, by identifying the patient's first eye in a first one of the images belonging to the first portion of the acquired images, and tracking the patient's first eye in images belonging the first portion of acquired images that were acquired subsequent to acquisition of the first one of the images belonging to the first portion of the acquired images.

For some applications, the computer processor is further configured to measure a pupillary response of a second eye of the patient to the light being directed toward the first eye, by identifying a pupil of the patient's second eye in images belonging to the acquired images that were acquired, respectively, prior to and subsequent to the light being directed toward the first eye.

There is further provided, in accordance with the invention, a method according to claim 4.

For some applications, identifying the patient's first eye within at least the first portion of the acquired images includes identifying the patient's first eye in a first one of the images belonging to the first portion of the acquired images, and tracking the patient's first eye in images belonging the first portion of acquired images that were acquired subsequent to acquisition of the first one of the images belonging to the first portion of the acquired images.

For some applications, the method further includes measuring a pupillary response of a second eye of the patient to the light being directed toward the first eye, by identifying a pupil of the patient's second eye in images belonging to the acquired images that were acquired, respectively, prior to and subsequent to the light being directed toward the first eye.

There is further provided, an apparatus including:
a light source;
at least one image-acquisition device configured to acquire a plurality of images of at least a portion of a face of a patient; and
at least one computer processor configured to:
   drive the light source to generate a flash of light; and
   measure a pupillary response of the patient, by identifying a pupil of an eye of the patient in images that were acquired, respectively, prior to and subsequent to the flash of light being generated.

There is additionally provided a method including:
acquiring a plurality of images of at least a portion of a face of a patient; and
using at least one computer processor:
   driving a light source to generate a flash of light; and
   measuring a pupillary response of the patient, by identifying a pupil of an eye of the patient in images that were acquired, respectively, prior to and subsequent to the flash of light being generated.

The present invention will be more fully understood from the following detailed description of applications thereof, taken together with the drawings, in which:
Fig. 1A is a schematic illustration of apparatus for automatically measuring a patient's respiratory rate, the apparatus including a sensor;
Fig. IB is a graph showing variation over time of pressure measured by a sensor as shown in Fig. 1A, the graph showing that pressure measured by the sensor varies with a patient's respiratory cycle;
Fig. 2 is a schematic illustration of a patient-testing station that includes apparatus for measuring a patient's systemic blood pressure; and
Fig. 3 is a schematic illustration of a patient-testing station that is configured to automatically measure a patient's pupillary light reflex, in accordance with some applications of the present invention.

### DETAILED DESCRIPTION OF EMBODIMENTS

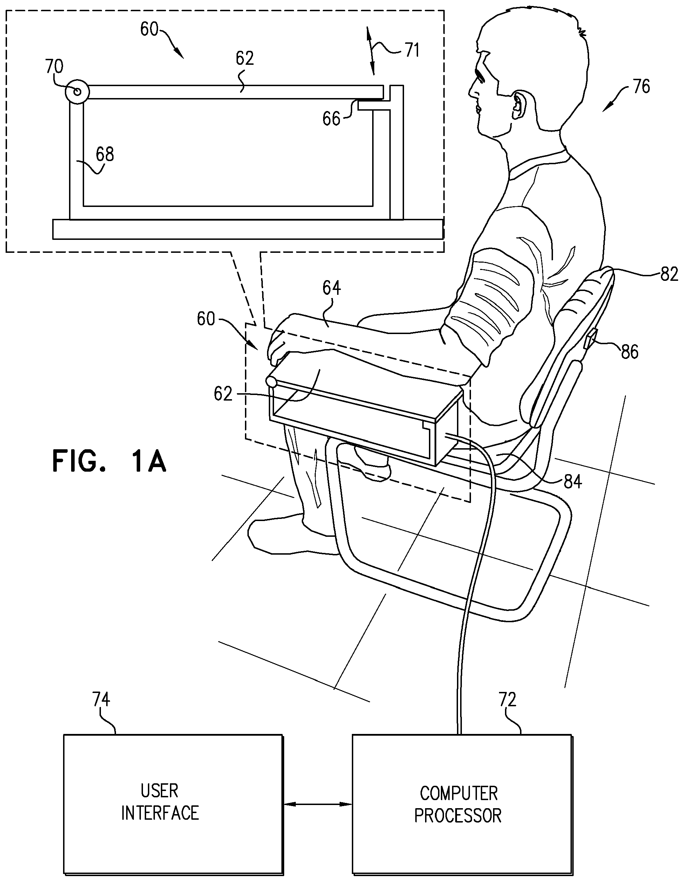

Reference is now made to Fig. 1A, which is a schematic illustration of apparatus 60 for automatically measuring a patient's respiratory rate. A surface 62 is configured to receive the patient's arm 64. A sensor 66 that is operatively coupled to the surface, detects movement of the surface, pressure exerted upon the surface, and/or force exerted upon the surface, and generates a sensor signal in response thereto.

For some applications, surface 62 is hingedly coupled to a supporting element 68, via a hinge 70. The surface is configured to rotate about the hinge (as indicated by arrow 71), in response to movement that the patient undergoes over the course of the patient's respiratory cycle. As described hereinabove, the sensor detects movement of the surface, pressure exerted upon the surface, and/or force exerted upon the surface, and generates a sensor signal in response thereto. Typically, due motion of the patient's arm resulting from the patient's respiratory cycle, the sensor signal contains a cyclical component that corresponds to the patient's respiratory cycle. A computer processor 72 is configured to receive the sensor signal, and to derive the patient's respiratory rate, at least partially based upon the received sensor signal.

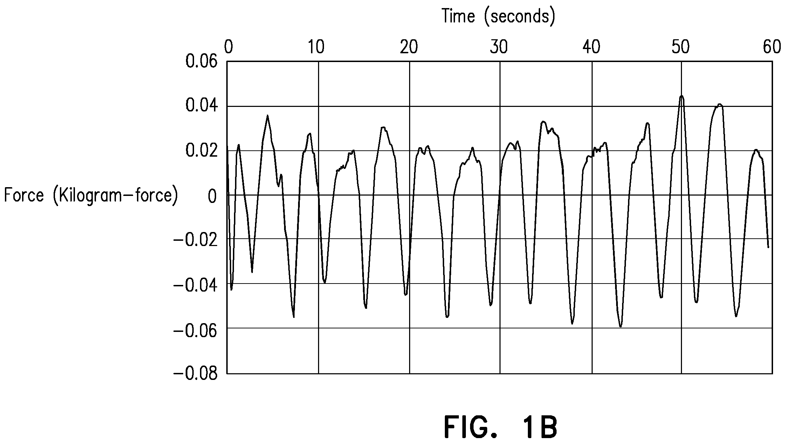

Reference is now made to Fig. 1B, which shows the variation over time of a sensor signal recorded using apparatus as generally described with reference to Fig. 1A, and using a force sensor as the sensor. As shown, the signal contains a cyclical component having an average period of approximately 4.5 seconds, there being approximately 13.5 cycles over the course of one minute. The aforementioned cyclical component corresponds to the respiratory cycle of the patient whose arm was placed on the surface during the recording of the data shown in Fig. 1B.

In accordance with the graph shown in Fig. 1B, for some applications, computer processor 72 derives the patient's respiratory cycle from the sensor signal by identifying a cyclical component within the signal and determining a parameter of the cyclical component, such as the mean frequency of the cyclical component, the mean period of the cyclical component, and/or the number of occurrences of the cyclical component over a given time interval (e.g., over the course of a minute). For some applications, the computer processor filters the sensor signal, in order to identify the cyclical component. For example, the computer processor may be configured to identify the cyclical component, by identifying a cyclical component having a minimum period of between 1 second and 3 seconds, and/or a maximum period of between 15 seconds and 30 seconds, e.g., a period of between 1 and 30 seconds, or between 3 and 15 seconds.

For some applications, the computer processor is further configured to determine additional parameters of the patient's respiratory cycle, by analyzing the cyclical component of the sensor signal. For example, the computer processor may be configured to determine a ratio (e.g., a mean ratio) between the duration of the patient's inspiration and the duration of the patient's expiration within the patient's respiratory cycle.

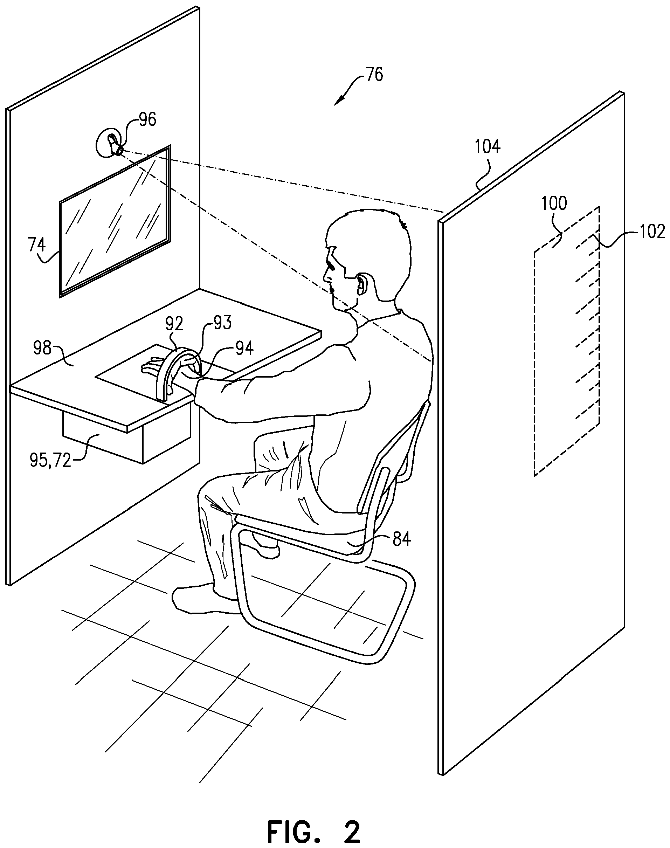

Typically, computer processor 72 communicates with a memory, and with a user interface 74. The patient typically sends instructions to the computer processor, via an input device of the user interface. For some applications, the user interface includes a keyboard, a mouse, a joystick, a touchscreen monitor (e.g., as shown in Fig. 2) a touchscreen device (such as a smartphone or a tablet computer), a touchpad, a trackball, a voice-command interface, and/or other types of input devices that are known in the art. Typically, the computer processor generates an output via an output device of the user interface. Further typically, the output device includes a display, such as a monitor, and the output includes an output that is displayed on the display. For some applications, the computer processor generates an output on a different type of visual, text, graphics, tactile, audio, and/or video output device, e.g., speakers, headphones, a smartphone, or a tablet computer. For example, the computer processor may generate an output on an output device associated with a given healthcare professional, and/or a given set of healthcare professionals. For some applications, as described hereinabove, user interface 74 includes both an input device and an output device (e.g., as shown in Fig. 2, which shows a touchscreen monitor). For some applications, the processor generates an output on a computer-readable medium (e.g., a non-transitory computer-readable medium), such as a disk, or a portable USB drive, and/or generates an output on a printer.

For some applications, respiratory-rate-measuring apparatus 60 comprises a portion of a patient-testing station 76.

For some applications, a compressible structure 82 is disposed upon a chair 84 upon which the patient sits. For example, the compressible structure may be disposed upon the back of the chair, such that the compressible structure is configured to be disposed between the patient's back and the back of the chair, when the patient sits on the chair. For some applications, the compressible structure includes an air-filled pillow, a gel-filled pillow, a balloon, and/or a similar structure. A sensor 86 is typically operatively coupled to the compressible structure, and is configured to detect movement of the compressible structure, pressure exerted upon the compressible structure, and/or force exerted upon the compressible structure, and to generate a sensor signal in response thereto.

The compressible structure is configured to become compressed and to expand, in response to movement that the patient undergoes over the course of the patient's respiratory cycle. As described hereinabove, the sensor detects movement of the compressible structure, pressure exerted upon the compressible structure, and/or force exerted upon the compressible structure, and generates a sensor signal in response thereto. Typically, due motion of the patient's torso, resulting from the patient's respiratory cycle, the sensor signal contains a cyclical component that corresponds to the patient's respiratory cycle. Computer processor 72 is configured to receive the sensor signal, and to derive the patient's respiratory rate, at least partially based upon the received sensor signal.

For some applications, computer processor 72 derives the patient's respiratory cycle from the sensor signal by identifying a cyclical component within the signal and determining a parameter of the cyclical component, such as the mean frequency of the cyclical component, the mean period of the cyclical component, and/or the number of occurrences of the cyclical component over a given time interval (e.g., over the course of a minute). For some applications, the computer processor filters the sensor signal, in order to identify the cyclical component. For example, the computer processor may be configured to identify the cyclical component, by identifying a cyclical component having a minimum period of between 1 second and 3 seconds, and/or a maximum period of between 15 second and 30 seconds, e.g., a period of between 1 and 30 seconds, or between 3 and 15 seconds. For some applications, the computer processor is further configured to determine additional parameters of the patient's respiratory cycle, by analyzing the cyclical component of the sensor signal. For example, the computer processor may be configured to determine a ratio (e.g., a mean ratio) between the duration of the patient's inspiration and the duration of the patient's expiration within the patient's respiratory cycle.

For some applications, the computer processor receives a sensor signal both from sensor 66 (which is operatively coupled to surface 62), as well as from sensor 86 (which is operatively coupled to compressible structure 82). For some such applications, the computer processor derives the patient's respiratory cycle from a combination of the first and second sensor signals. For example, the computer processor may identify a cyclical component in one of the sensor signals, and may determine that that cyclical component does not correspond to respiration of the patient, by comparing that sensor signal to the other sensor signal. Or, for example, if movement of the patient's arm as a result of the respiratory cycle is not sufficiently strong to be detected by sensor 66, then the computer processor may nevertheless detect the patient's respiratory cycle, based upon the sensor signal from sensor 86, or vice versa.

It is noted that even if the amplitude of the cyclical component of the sensor signals is low, the computer processor is typically configured to detect the cyclical component, e.g., by identifying a component of the sensor signal that is cyclical and that has a frequency within a given range, as described hereinabove.

For some applications, the computer processor generates an output that is indicative of the determined respiratory rate. Alternatively or additionally, the computer processor determines the value of a different physiological parameter and or diagnoses the patient as suffering from a given condition, at least partially based upon the determined respiratory rate, and generates an output that is indicative of the other physiological parameter, and/or the diagnosis (where any diagnostic method is not part of the claimed invention). . Further alternatively or additionally, the computer processor triages the patient (and generates a corresponding output), and/or generates an alert at least partially based upon the determined respiratory rate.

Reference is now made to Fig. 2, which is a schematic illustration of patient-testing station 76, the patient-testing station including apparatus for measuring a patient's systemic blood pressure, in accordance with some applications of the present disclosure. Typically, the patient-testing station includes pressure-sensing apparatus 92, which is placed in contact with a portion of the patient's body, e.g., the patient's wrist 94, as shown in Fig. 1A. For some applications, the pressure-sensing apparatus includes a compressible portion 93 (e.g., an inflatable cuff, or sleeve) configured to be placed in contact with the portion of the patient's body, and a pressure sensor 95 configured to measure blood pressure of the portion of the patient's body, by detecting pressure applied to the compressible portion by the portion of the patient's body. A camera 96 is typically configured to acquire one or more images of the patient. For some applications, computer processor 72 estimates a location of patient's heart, by analyzing the one or more images of the patient.

The computer processor typically estimates a difference in height between the portion of the patient's body that is in contact with the pressure-sensing apparatus (e.g., the patient's wrist) and the estimated location of the patient's heart, and generates an output in response thereto. Typically, the computer processor determines the patient's systemic blood pressure, based upon the blood pressure of the portion of the patient's body (e.g., the patient's wrist) measured by the pressure-sensing apparatus, and the estimated difference in height between the portion of the patient's body that is in contact with the pressure-sensing apparatus and the estimated location of the patient's heart. For example, the computer processor may apply a compensation to the pressure measured by the pressure-sensing apparatus, to account for the estimated difference in height between the portion of the patient's body that is in contact with the pressure-sensing apparatus and the estimated height of the patient's heart.

For some applications, the computer processor generates an output that is indicative of the determined systemic blood pressure. Alternatively or additionally, the computer processor determines the value of a different physiological parameter and or diagnoses the patient as suffering from a given condition, at least partially based upon the determined systemic blood pressure, and generates an output that is indicative of the other physiological parameter, and/or the diagnosis. Further alternatively or additionally, the computer processor triages the patient (and generates a corresponding output), and/or generates an alert at least partially based upon the determined systemic blood pressure.

For some applications, the computer processor generates the output at least partially by automatically reducing a height difference between at least a portion of the pressure-sensing apparatus (e.g., the compressible portion, such as the cuff described hereinabove) and the estimated location of the heart. For example, as shown in Fig. 2, patient-testing station may include chair 84, upon which the patient sits during the blood pressure measurement. The computer processor may automatically reduce the height difference between the pressure-sensing apparatus and the estimated location of the heart, by automatically adjusting a height of the chair. Alternatively or additionally, the computer processor may automatically reduce the height difference between the pressure-sensing apparatus and the estimated location of the patient's heart, by automatically adjusting a height of the pressure-sensing apparatus. For example, the pressure-sensing apparatus may include a cuff that is disposed upon a surface 98 (as shown), and the computer processor may automatically adjust the height of the surface.

For some applications, a surface 100 having markings 102 thereon is configured to be disposed in a vicinity of the patient during acquisition of the one or more images of the patient. For example, the surface may be disposed upon a wall 104 of patient-testing station 76 that is behind the patient's back. For some applications, the computer processor estimates the location of the patient's heart by identifying at least some of the markings within the one or more images of the patient. For example, the computer processor may estimate a location of the patient's heart within the patient's body by analyzing the images of the patient. Using the markings as a reference, the computer processor may then determine the height of the patient's heart with respect to the patient-testing station, or a portion thereof. For some applications, the computer processor estimates the difference in height between the portion of the patient's body that is in contact with the pressure sensor and the estimated location of the patient's heart, by identifying at least some of the markings within the one or more images of the patient, using a generally similar technique.

For some applications, computer processor 72 is in-built to the patient-testing station, as shown. As described hereinabove, typically, the computer processor communicates with a memory, and with a user interface 74. The patient typically sends instructions to the computer processor, via an input device of the user interface. For some applications, the user interface includes a keyboard, a mouse, a joystick, a touchscreen device (such as a smartphone or a tablet computer), a touchpad, a trackball, a voice-command interface, and/or other types of input devices that are known in the art. Typically, the computer processor generates an output via an output device of the user interface. Further typically, the output device includes a display, such as a monitor, as shown, and the output includes an output that is displayed on the display. For some applications, the computer processor generates an output on a different type of visual, text, graphics, tactile, audio, and/or video output device, e.g., speakers, headphones, a smartphone, or a tablet computer. For example, the computer processor may generate an output on an output device associated with a given healthcare professional, and/or a given set of healthcare professionals. For some applications, as described hereinabove, user interface 74 includes both an input device and an output device. For example, as shown in Fig. 2, the user interface may include a touchscreen monitor. For some applications, the processor generates an output on a computer-readable medium (e.g., a non-transitory computer-readable medium), such as a disk, or a portable USB drive, and/or generates an output on a printer.

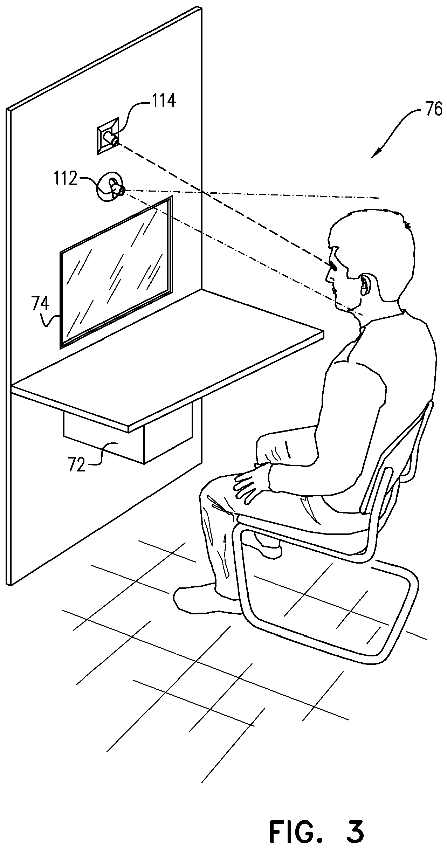

Reference is now made to Fig. 3, which is a schematic illustration of patient-testing station 76, the patient-testing station being configured to automatically measure a patient's pupillary light reflex.

At least one image-acquisition device 112 acquires a plurality of images of at least a portion of the patient's face. For example, the image-acquisition device may be a video camera that is configured to acquire images of at least a portion of the patient's face that includes at least one of the patient's eyes. Computer processor 72 identifies a first eye of the patient within at least a first portion of the acquired images. For some applications, the computer processor identifies the pupil of the first eye within a first portion of the acquired images. In response to identifying the first eye (and/or the pupil thereof), the computer drives a moveable light source 114 (e.g., a laser and/or a broadband light source) to direct light toward the patient's first eye (and/or the pupil thereof). For example, as shown in Fig. 3, the computer processor may identify the patient's left eye within the first portion of the acquired images and may drive the moveable light source to direct light toward the patient's left eye. For some applications, the moveable light source is configured to be moved automatically, and the movability of the light source typically is in at least two degrees of freedom, such that light from the light source can be directed anywhere upon the patient's face.

The computer processor measures the pupillary light reflex of the first eye to the light being directed toward the first eye, by identifying a pupil of the patient's first eye in images belonging to the first portion of the acquired images that were acquired, respectively, prior to and subsequent to the light being directed toward the first eye. For example, in a first image that was acquired prior to the light being directed toward the patient's left eye, the computer processor may identify the pupil of the patient's left eye and may determine that the pupil has a diameter of x mm. The computer processor may then identify the pupil of the patient's left eye within images that were acquired subsequent to the light being directed toward the patient's left eye, and may thereby determine in which of those images the diameter of the pupil has decreased relative to x mm, and/or in which of those images the diameter of the pupil has decreased by more than a threshold amount and/or more than a threshold percentage, relative to x mm.

For some applications, computer processor 72 identifies the patient's first eye (and/or the pupil thereof) within at least the first portion of the acquired images, by identifying the patient's first eye (and/or the pupil thereof) in a first one of the images belonging to the first portion of the acquired images, and tracking the patient's first eye (and/or the pupil thereof) in images belonging the first portion of acquired images that were acquired subsequent to acquisition of the first one of the images belonging to the first portion of the acquired images. For some applications, in order to track the patient's eye (and/or the pupil thereof), the computer processor drives a light source (e.g., light source 114 and/or a different light source) to direct infrared and/or near-infrared non-collimated light generally toward the region in which the first eye is disposed, or generally in the direction of the patient's face. The light is configured to reflect from the patient's cornea. By identifying the reflected light in the images, the computer processor determines the location of the patient's eye (and/or the pupil thereof).

For some applications, the computer processor is further configured to identify the pupil of the patient's second eye within at least some of the acquired images. By way of example, the computer processor may be configured to identify the pupil of the patient's right eye within some of the acquired images. For some applications, the computer processor identifies the pupil of the patient's second eye within the second portion of the acquired images, by identifying the pupil of the patient's second eye in a first one of the images belonging to a second portion of the acquired images, and tracking the pupil of the patient's second eye in images belonging the second portion of acquired images that were acquired subsequent to acquisition of the first one of the images belonging to the second portion of the acquired images, e.g., using generally similar techniques to those described hereinabove. It is noted that for some applications, both of the patient's eyes (and/or pupils thereof) are identified in a single portion of the acquired images. In such cases, the "first" and "second" portions of images described hereinabove, comprise a single portion of images.

In accordance with the invention, the computer processor measures the patient's consensual pupillary reflex by measuring a pupillary light reflex of the second eye, to the light being directed toward the first eye, by identifying the pupil of the patient's second eye in images that were acquired, respectively, prior to and subsequent to the light being directed toward the first eye. For example, in a first image that was acquired prior to the light being directed toward the patient's left eye, the computer processor may identify the pupil of the patient's right eye and may determine that the pupil has a diameter of x mm. The computer processor may then identify the pupil of the patient's right eye within images that were acquired subsequent to the light being directed toward the patient's left eye, and may thereby determine in which of those images the diameter of the pupil has decreased relative to x mm, and/or in which of those images the diameter of the pupil has decreased by more than a threshold amount and/or more than a threshold percentage, relative to x mm.

For some applications (not according to the invention), the computer processor determines the patient's pupillary light reflex in a generally similar manner to that described hereinabove. However, rather than identifying the patient's first eye (and/or the pupil thereof) and directing light toward the first eye (and/or the pupil thereof), the computer processor drives the light source to generate a flash of light that is not specifically directed toward the patient's first eye (and/or the pupil thereof). The computer processor identifies pupils of the patient's first eye and/or second eye in images acquired before and after the generation of the flash of light, and thereby determines the patient's pupillary light reflex in a generally similar manner to that described hereinabove.

For some applications, the computer processor diagnoses a condition of the patient, generates an alert, and/or generates a different output at least partially based upon the pupillary light reflex of the first eye, and/or the second eye. For some applications, the computer processor generates an output that is indicative of the determined pupillary light reflex of the first eye, and/or the second eye. Alternatively or additionally, the computer processor determines the value of a different physiological parameter and or diagnoses the patient as suffering from a given condition, at least partially based upon the determined pupillary light reflex of the first eye, and/or the second eye, and generates an output that is indicative of the other physiological parameter, and/or the diagnosis. Further alternatively or additionally, the computer processor triages the patient (and generates a corresponding output), and/or generates an alert at least partially based upon the determined pupillary light reflex of the first eye, and/or the second eye.

For some applications, computer processor 72 is in-built to the patient-testing station, as shown. As described hereinabove, typically, the computer processor communicates with a memory, and with a user interface 74. The patient typically sends instructions to the computer processor, via an input device of the user interface. For some applications, the user interface includes a keyboard, a mouse, a joystick, a touchscreen device (such as a smartphone or a tablet computer), a touchpad, a trackball, a voice-command interface, and/or other types of input devices that are known in the art. Typically, the computer processor generates an output via an output device of the user interface. Further typically, the output device includes a display, such as a monitor, as shown, and the output includes an output that is displayed on the display. For some applications, the computer processor generates an output on a different type of visual, text, graphics, tactile, audio, and/or video output device, e.g., speakers, headphones, a smartphone, or a tablet computer. For example, the computer processor may generate an output on an output device associated with a given healthcare professional, and/or a given set of healthcare professionals. For some applications, as described hereinabove, user interface 74 includes both an input device and an output device. For example, as shown in Fig. 2, the user interface may include a touchscreen monitor. For some applications, the processor generates an output on a computer-readable medium (e.g., a non-transitory computer-readable medium), such as a disk, or a portable USB drive, and/or generates an output on a printer.

For some applications, the apparatus and methods described hereinabove with reference to Figs. 1A, 1B, 2 and/or 3 are used in conjunction with apparatus and methods described in WO 18/220565 to Amir. For example, the apparatus and methods described herein may be used in an emergency-room setting, in order to triage and/or diagnose patients. Alternatively, the methods and apparatus described with reference to Figs. 1A, 1B, 2 and/or 3 may be used in a different setting. For example, the methods and apparatus described with reference to Figs. 1A, 1B, 2 and/or 3 may be used for determining a patient's respiratory cycle, systemic blood pressure, and/or pupillary reflex, in a non-hospital setting, e.g., in a physician's office, in a pharmacy, in a home setting, and/or in a laboratory. Alternatively or additionally, the methods and apparatus described herein may be used for determining a patient's respiratory cycle, systemic blood pressure, and/or pupillary reflex, in a hospital setting, but outside of an emergency-room setting, e.g., for monitoring in-patients and/or out-patients within the hospital.

Applications of the invention described herein can take the form of a computer program product accessible from a computer-usable or computer-readable medium (e.g., a non-transitory computer-readable medium) providing program code for use by or in connection with a computer or any instruction execution system, such as computer processor 72. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W) and DVD.

A data processing system suitable for storing and/or executing program code will include at least one processor (e.g., computer processor 72) coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an object-oriented programming language such as Java, Smalltalk, C++ or the like and conventional procedural programming languages, such as the C programming language or similar programming languages.

It will be understood that the algorithms described herein, can be implemented by computer program instructions. These computer program instructions may be provided to a processor of a general-purpose computer, special purpose computer, or other programmable data processing apparatus to produce a machine, such that the instructions, which execute via the processor of the computer (e.g., computer processor 72) or other programmable data processing apparatus, create means for implementing the functions/acts specified in the algorithms described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the algorithms. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the algorithms described in the present application.

Computer processor 72 is typically a hardware device programmed with computer program instructions to produce a special purpose computer. For example, when programmed to perform the algorithms described with reference to the Figures, computer processor 72 typically acts as a special purpose patient-analysis computer processor. Typically, the operations described herein that are performed by computer processor 72 transform the physical state of a memory, which is a real physical article, to have a different magnetic polarity, electrical charge, or the like depending on the technology of the memory that is used. For some applications, operations that are described as being performed by a computer processor are performed by a plurality of computer processors in combination with each other.

The present application is related to International Application PCT/IB2018/053869 to Amir (published as WO 18/220565), filed May 31, 2018.

It will be appreciated by persons skilled in the art that the present invention is not limited to what has been particularly shown and described hereinabove. Rather, the scope of the present invention is defined solely by the claims.

## Claims

1. Apparatus comprising:
a moveable light source (114);
at least one image-acquisition device (112) configured to acquire a plurality of images of at least a portion of a face of a patient; and
at least one computer processor (72) configured to:
identify a first eye of the patient within at least a first portion of the acquired images;
in response thereto, drive the light source (114) to direct light toward the patient's first eye; and
measure a consensual pupillary response of a second eye of the patient to the light being directed toward the first eye, by identifying a pupil of the patient's second eye in images belonging to the acquired images that were acquired, respectively, prior to and subsequent to the light being directed toward the first eye.

2. The apparatus according to claim 1, wherein the at least one computer processor (72) is configured to identify the patient's first eye within at least the first portion of the acquired images, by identifying the patient's first eye in a first one of the images belonging to the first portion of the acquired images, and tracking the patient's first eye in images belonging the first portion of acquired images that were acquired subsequent to acquisition of the first one of the images belonging to the first portion of the acquired images.

3. The apparatus according to claim 1 or claim 2, wherein the computer processor (72) is further configured to measure a pupillary response of the first eye of the patient to the light being directed toward the first eye, by identifying a pupil of the patient's first eye in images belonging to the first portion of the acquired images that were acquired, respectively, prior to and subsequent to the light being directed toward the first eye.

4. A method comprising:
acquiring a plurality of images of at least a portion of a face of a patient; and
using at least one computer processor (72):
identifying a first eye of the patient within at least a first portion of the acquired images;
in response thereto, driving a moveable light source (114) to direct light toward the patient's first eye; and
measuring a consensual pupillary response of a second eye of the patient to the light being directed toward the first eye, by identifying a pupil of the patient's second eye in images belonging to the acquired images that were acquired,
respectively, prior to and subsequent to the light being directed toward the first eye.

5. The method according to claim 4, wherein identifying the patient's first eye within at least the first portion of the acquired images comprises identifying the patient's first eye in a first one of the images belonging to the first portion of the acquired images, and tracking the patient's first eye in images belonging the first portion of acquired images that were acquired subsequent to acquisition of the first one of the images belonging to the first portion of the acquired images.

6. The method according to claim 4 or claim 5, further comprising measuring a pupillary response of the first eye of the patient to the light being directed toward the first eye, by identifying a pupil of the patient's first eye in images belonging to the first portion of the acquired images that were acquired, respectively, prior to and subsequent to the light being directed toward the first eye.

## Patentansprüche

1. Apparat, umfassend:
eine bewegbare Lichtquelle (114);
wenigstens eine Bild-Erfassung-Vorrichtung (112), die dazu eingerichtet ist, eine Mehrzahl von Bildern wenigstens eines Abschnitts eines Gesichts eines Patienten zu erfassen; und
wenigstens einen Computerprozessor (72), der dazu eingerichtet ist:
ein erstes Auge des Patienten innerhalb wenigstens eines ersten Abschnitts der erfassten Bilder zu identifizieren;
als Reaktion darauf, die Lichtquelle (114) anzutreiben, um Licht in Richtung des ersten Auges des Patienten zu richten; und
eine gleichsinnige Pupillenreaktion eines zweiten Auges des Patienten auf das in Richtung des ersten Auges gerichtete Licht durch Identifizieren einer Pupille des zweiten Auges des Patienten in Bildern zu messen, die zu den erfassten Bildern gehören, die bevor beziehungsweise nachfolgend, nachdem das Licht in Richtung des ersten Auges gerichtet ist, erfasst wurden.

2. Apparat nach Anspruch 1, wobei der wenigstens eine Computerprozessor (72) dazu eingerichtet ist, das erste Auge des Patienten innerhalb wenigstens des ersten Abschnitts der erfassten Bilder durch Identifizieren des ersten Auges des Patienten in einem ersten der Bilder, die zu dem ersten Abschnitt der erfassten Bilder gehören, und Verfolgen des ersten Auges des Patienten in Bildern zu identifizieren, die zu dem ersten Abschnitt der erfassten Bilder gehören, die einer Erfassung des ersten der Bilder, die zu dem ersten Abschnitt der erfassten Bilder gehören, nachfolgend erfasst wurden.

3. Apparat nach Anspruch 1 oder Anspruch 2, wobei der Computerprozessor (72) ferner dazu eingerichtet ist, eine Pupillenreaktion des ersten Auges des Patienten auf das Licht, das in Richtung des ersten Auges gerichtet wird, durch Identifizieren eine Pupille des ersten Auges des Patienten in Bildern zu messen, die zu dem ersten Abschnitt der erfassten Bilder gehören, die bevor beziehungsweise nachfolgend, nachdem das Licht in Richtung des ersten Auges gerichtet ist, erfasst wurden.

4. Verfahren, umfassend:
Erfassen einer Mehrzahl von Bildern wenigstens eines Abschnitts eines Gesichts eines Patienten; und
Verwenden wenigstens eines Computerprozessors (72):
Identifizieren eines ersten Auges des Patienten innerhalb wenigstens eines ersten Abschnitts der erfassten Bilder;
als Reaktion darauf, Antreiben einer bewegbaren Lichtquelle (114), um Licht in Richtung des ersten Auges des Patienten zu richten; und
Messen einer gleichsinnigen Pupillenreaktion eines zweiten Auges des Patienten auf das in Richtung des ersten Auges gerichtete Licht, durch Identifizieren einer Pupille des zweiten Auges des Patienten in Bildern, die zu den erfassten Bildern gehören, die bevor beziehungsweise nachfolgend, nachdem das Licht in Richtung des ersten Auges gerichtet wurde, erfasst wurden.

5. Verfahren nach Anspruch 4, wobei das Identifizieren des ersten Auges des Patienten innerhalb wenigstens des einen ersten Abschnitts der erfassten Bilder ein Identifizieren des ersten Auges des Patienten in einem ersten der Bilder, die zu dem ersten Abschnitt der erfassten Bilder gehören, und ein Verfolgen des ersten Auges des Patienten in Bildern, die zu dem ersten Abschnitt von erfassten Bildern gehören, die einer Erfassung des ersten der Bilder, die zu dem ersten Abschnitt der erfassten Bilder gehören, nachfolgend erfasst wurden, umfasst.

6. Verfahren nach Anspruch 4 oder Anspruch 5, ferner umfassend Messen einer Pupillenreaktion des ersten Auges des Patienten auf das Licht, das in Richtung des ersten Auges gerichtet wird, durch Identifizieren einer Pupille des ersten Auges des Patienten in Bildern, die zu dem ersten Abschnitt der erfassten Bilder gehören, die bevor beziehungsweise nachfolgend, nachdem das Licht in Richtung des ersten Auges gerichtet wurde, erfasst wurden.

## Revendications

1. Appareil comprenant :
une source de lumière mobile (114) ;
au moins un dispositif d'acquisition d'image (112) configuré pour acquérir une pluralité d'images d'au moins une partie du visage d'un patient ; et
au moins un processeur informatique (72) configuré pour :
identifier un premier œil du patient dans au moins une première partie des images acquises ;
en réponse à cela, entraîner la source de lumière (114) à diriger de la lumière vers le premier œil du patient ; et
mesurer une réponse pupillaire consensuelle d'un second œil du patient à la lumière dirigée vers le premier œil, en identifiant une pupille du second œil du patient dans des images appartenant aux images acquises qui ont été acquises, respectivement, avant et après que la lumière soit dirigée vers le premier œil.

2. Appareil selon la revendication 1, dans lequel le au moins un processeur informatique (72) est configuré pour identifier le premier œil du patient dans au moins la première partie des images acquises, en identifiant le premier œil du patient dans une première des images appartenant à la première partie des images acquises, et en suivant le premier œil du patient dans des images appartenant à la première partie des images acquises qui ont été acquises suite à une acquisition de la première des images appartenant à la première partie des images acquises.

3. Appareil selon la revendication 1 ou la revendication 2, dans lequel le processeur informatique (72) est en outre configuré pour mesurer une réponse pupillaire du premier œil du patient à la lumière dirigée vers le premier œil, en identifiant une pupille du premier œil du patient dans des images appartenant à la première partie des images acquises qui ont été acquises, respectivement, avant et après que la lumière soit dirigée vers le premier œil.

4. Procédé, comprenant les étapes consistant à :
acquérir une pluralité d'images d'au moins une partie du visage d'un patient ; et
utiliser au moins un processeur informatique (72) :
identifiant un premier œil du patient dans au moins une première partie des images acquises ;
en réponse à cela, entraînant une source de lumière mobile (114) à diriger de la lumière vers le premier œil du patient ; et
mesurant une réponse pupillaire consensuelle d'un second œil du patient à la lumière dirigée vers le premier œil, en identifiant une pupille du second œil du patient dans des images appartenant aux images acquises qui ont été acquises, respectivement, avant et après que la lumière soit dirigée vers le premier œil.

5. Procédé selon la revendication 4, dans lequel l'identification du premier œil du patient dans au moins la première partie des images acquises comprend une identification du premier œil du patient dans une première des images appartenant à la première partie des images acquises, et un suivi du premier œil du patient dans des images appartenant à la première partie des images acquises qui ont été acquises suite à une acquisition de la première des images appartenant à la première partie des images acquises.

6. Procédé selon la revendication 4 ou la revendication 5, comprenant en outre une mesure d'une réponse pupillaire du premier œil du patient à la lumière dirigée vers le premier œil, en identifiant une pupille du premier œil du patient dans des images appartenant à la première partie des images acquises qui ont été acquises, respectivement, avant et après que la lumière soit dirigée vers le premier œil.
